(19) Europäisches Patentamt
European Patent Office
Office européen des brevets

(11) EP 0 723 532 B1

(12) # EUROPEAN PATENT SPECIFICATION

(45) Date of publication and mention of the grant of the patent:
**29.07.1998 Bulletin 1998/31**

(21) Application number: **94930163.4**

(22) Date of filing: **10.10.1994**

(51) Int. Cl.$^6$: **C07C 51/09**

(86) International application number:
**PCT/EP94/03357**

(87) International publication number:
**WO 95/10499 (20.04.1995 Gazette 1995/17)**

(54) **PROCESS FOR RECOVERING DICARBOXYLIC ACID WITH REDUCED IMPURITIES FROM POLYESTER POLYMER**

VERFAHREN ZUR RÜCKGEWINNUNG VON VERMINDERTE VERUNREINIGUNGEN ENTHALTENDE DICARBONSÄURE AUS POLYESTERPOLYMERISAT

PROCEDE DE RECUPERATION D'ACIDE DICARBOXYLIQUE A TENEUR REDUITE EN IMPURETES, A PARTIR D'UN POLYMERE DE POLYESTER

(84) Designated Contracting States:
**AT BE CH DE FR GB IT LI LU NL**

(30) Priority: **14.10.1993 US 136636**

(43) Date of publication of application:
**31.07.1996 Bulletin 1996/31**

(73) Proprietor:
**SHELL INTERNATIONALE RESEARCH MAATSCHAPPIJ B.V.**
**2596 HR Den Haag (NL)**

(72) Inventor: **ROLLICK, Kevin, Louis**
**Munroe Falls, OH 44262 (US)**

(56) References cited:
**GB-A- 1 476 539**
**US-A- 5 278 256**

- **PATENT ABSTRACTS OF JAPAN vol. 5, no. 194 (C-82) (866) 10 December 1981 & JP,A,56 113 738 (TORAY K.K.) 7 September 1981**

EP 0 723 532 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Xerox (UK) Business Services
2.16.3/3.4

## Description

This invention relates to a process for depolymerizing polyester polymers by hydrolysis. More particularly, this invention relates to a process for recovering terephthalic acid and ethylene glycol from polyethylene terephthalate via base hydrolysis.

The hydrolysis of high molecular weight polyesters, such as polyethylene terephthalate (PET) is well known in the art; see, for example, British Patent Specification No. 822,834. The hydrolysis process is essentially a reversal of the polymerization process, and, for PET, results in the production of terephthalic acid and ethylene glycol.

As for all polymer recycling, post-consumer PET recycle streams are contaminated by a variety of substances which must be removed or minimized to facilitate recovery and reuse. For PET containers, residue from the container's original contents is present, along with the other plastics, resins, and glues used in the container fabrication process. Catalyst residues and additives (such as dyes and pigments) from production of the PET are present in the polymer itself. In addition, dirt and oil picked up along the use and recycle route are present in varying amounts. Recycling processes currently in use vary in their ability to remove these contaminants. Some require costly purification steps which result in the recycle products being significantly more expensive than the virgin materials. Base hydrolysis may include a carbon adsorption bed, or a step to extract with alcohol, to remove coloured impurities; see, for example, European Patent Specification No. 497,662.

In GB-A 1 476 539 there is disclosed a process for recovering terephthalic acid from light sensitive silver emulsion polyester film material, comprising the oxidation of the polyester in an aqueous alkaline medium wherein the silver component remains in the solid phase, separating the solid phase, and acidifying the liquid phase to precipitate the terephthalic component dissolved therein.

The present invention provides a process for recovering dicarboxylic acid from a polyester feed stream which comprises:

(a) contacting the polyester with an alkali metal hydroxide and a quaternary ammonium hydroxide or a nonionic surfactant and, if necessary, water to form a hydrolyzed mixture containing an alkali metal carboxylic acid salt and a diol or glycol,
(b) oxidizing the hydrolyzed mixture through addition of an oxidizing agent, to form an oxidized mixture containing insoluble impurities,
(c) removing the insoluble impurities from the oxidized mixture,
(d) acidifying the oxidized mixture to form an acidified mixture from which the dicarboxylic acid is precipitated, and
(e) recovering dicarboxylic acid from the acidified mixture.

The subject invention is an improved process for depolymerizing a polyester polymer back into its raw materials, a dicarboxylic acid and a glycol. The rate of the hydrolysis reaction is increased by the addition of a small amount of a quaternary ammonium hydroxide, or a nonionic surfactant. A further improvement provided by the subject invention is addition of an oxidation step, to oxidize impurities present in the hydrolyzed mixture, the oxidation step preferably being conducted by sparging air into the hydrolyzed mixture. The impurities are oxidized into insoluble impurities, which may be separated by filtration or other conventional means.

More preferably, the subject invention is an improved process for depolymerizing PET back into its raw materials, terephthalic acid and ethylene glycol, and recovering such raw materials. The invention employs a base hydrolysis process, wherein the PET is contacted with an aqueous solution of an alkali metal hydroxide to form a hydrolyzed solution containing an alkali metal terephthalate and ethylene glycol. The oxidized mixture, after separation of the impurities, is acidified with a mineral acid to precipitate terephthalic acid, which is separated by filtration or other conventional means. Ethylene glycol is recovered by conventional methods at any point in the overall process scheme. Removal of impurities results in a purer, polymer grade terephthalic acid product, of higher value and more readily recycled into new PET materials. Thus, the proposed process results in a terephthalic acid product of high purity, suitable for reuse in making PET, without costly purification steps.

The feed streams utilized in practicing this invention contain polyesters, such as recycle PET feed streams. However, other plastics such as polyvinyl chloride, high density polyethylene, low density polyethylene, polypropylene, and ethylene vinyl acetates can also be present in a recycle stream. The polyesters include a variety of condensation polymers formed by the combination of a dicarboxylic acid or diester thereof and a dihydric alcohol or glycol. PET is the most common polyester currently recycled, and therefore this text concentrates on the application of the present process to the recycling of PET; however, the subject invention is applicable to, and is intended to encompass, the treatment of a variety of polyesters, indeed even the purification of prepared polyester as well as the treatment of recycled polyester.

PET in a recycle stream is typically comprised of repeat units which are derived from terephthalic acid or a diester thereof and ethylene glycol (1,2-ethanediol). However, it is understood that the PET can also be modified with small

amounts of other monomers. Such modified PET can contain small amounts of repeat units which are derived from diacids other than terephthalic acid and/or glycols in addition to ethylene glycol. For instance, small amounts of isophthalic acid or a naphthalene dicarboxylic acid can be used in the diacid component utilized in preparing the PET. PET which has been modified with a small amount of a diol containing from 3 to 8 carbon atoms is also representative of such a modified PET. For instance, a small amount of 1,4-butanediol can be utilized in the glycol component used in preparing the modified PET. Normally, no more than about 15 weight percent of the repeat units in such modified PET will be comprised of diacids or diols other than a terephthalic acid and ethylene glycol. Other polyesters include polypropylene terephthalate (PPT), polybutylene terephthalate (PBT), polyethylene naphthalate (PEN), and polybutylene naphthalate (PBN). PPT is derived from terephthalic acid or a diester thereof and propylene glycol (1,3-propanediol). PBT is derived from terephthalic acid or a diester thereof and 1,4-butanediol. PEN is derived from a naphthalene dicarboxylic acid or a diester thereof, such as 2,6-naphthalene dicarboxylic acid, and ethylene glycol (1,2-ethanediol). PBN is derived from a naphthalene dicarboxylic acid or a diester thereof, such as 2,6-naphthalene dicarboxylic acid, and 1,4-butanediol.

PET in a recycle feed stream is primarily derived from PET compounded into bottles and other containers, but may also be derived from other applications, such as fibres and film. PET articles which are being recycled are typically mechanically converted into "flakes" for further processing. For instance, PET beverage bottles are typically ground into flakes which have a cross-sectional area in the range of from 4 $mm^2$ to 100 $mm^2$ It is more typical for such flakes to have an area in the range of from 15 $mm^2$ to 65 $mm^2$ The thickness of the flakes varies with the wall thickness of the bottles which are being recycled. The PET can contain catalyst deactivators, such as phosphates, and other additives, such as impact modifiers, process aids, fillers, pigments and dyes (organic and inorganic), ultra-violet light stabilizers, and antidegradants.

The base hydrolysis process of the subject invention is directly applicable to such polyester flake. As an alternative, the polyester may be partially depolymerized into oligomers (polymer chains with only a small number of monomer units) prior to base hydrolysis. Standard depolymerization techniques can be used in this step; for example, the procedures described in U.S. Patent Specification No. 3,703,488 and U.S. Patent Specification No. 3,884,850 may be used in depolymerizing PET. It is generally desirable to depolymerize PET by adding in the range of from 2 weight percent to 50 weight percent ethylene glycol to a PET feed stream and heating the resulting PET/ethylene glycol mixture at a temperature in the range of from 180°C to 310°C to cause depolymerization. It is preferred to use a temperature in the range of from 220°C to 270°C and it is generally preferred to use in the range of from 7 weight percent to 20 weight percent ethylene glycol. Depolymerization procedures which use other polar solvents capable of depolymerizing polyesters, such as propylene glycol or butanediol, water, or acid in place of ethylene glycol can also be used.

For PET specifically the presence of impurities from recovered raw materials is a significant problem. Impurities present in the PET polymer itself, in addition to impurities present in the post-consumer PET recycle stream, are a source of colour in the recovered terephthalic acid. Because of certain of the PET end-uses, it is highly desirable for the recovered terephthalic acid to be as white as possible, since any colour present in the terephthalic acid will be transferred to the PET product when the terephthalic acid is reused to make PET. To produce clear bottles, for example, the PET must be a colourless white polymer.

The colour of a terephthalic acid product may be measured by various conventional methods, such as spectrophotometry, which is explained below. Substances which appear coloured in transmitted light (a glass or a bottle, for example) do so because they are absorbing light in a complementary portion of the spectrum. For example, an item which appears yellow is absorbing violet/blue light. The degree of yellowness can be quantified by passing violet/blue light through a sample and comparing the intensity of the light before ($I_o$) and after (I) passing through the sample. Instruments which are designed for this task are referred to as spectrophotometers - in this case, visible or UV-visible spectrophotometers.

In practice the ratio of the intensities of the light before and after passing through the sample is sometimes reported:

$$T = I/I_o$$

Alternatively, the logarithm of the reciprocal of the transmittance (T) or the absorbance (A) is often reported:

$$A = -\log T = \log (1/T) = \log (I_o/I)$$

The absorbance at any wavelength is directly related to the number of absorbing molecules or ions by the following equation commonly known as Beer's law:

$$A = (a)\ (b)\ (c)$$

where a is the absorptivity of the absorbing substance, b is the path length of the solution being measured and c is the concentration of the absorbing substance in solution. The constant a is an intrinsic property of the substance in a given solvent at a given wavelength. In practice, b is held constant by use of a sample holder of given dimensions - most often 1 cm. From this discussion it follows that an increase in the absorbance of a solution at a given wavelength is due either to an increase in the concentration of the absorbing species or the introduction of a new species with a higher absorptivity. Also, the higher the absorbance, the more light is being absorbed at that wavelength and so the more intense will be complementary colour of the solution.

In the base hydrolysis process, polyester, e.g. PET, is typically contacted with an aqueous solution of an alkali metal hydroxide to form a hydrolyzed mixture containing an alkali metal salt, e.g. a terephthalate, and diol or glycol, e.g. ethylene glycol. The polyester dissolves as it depolymerizes in the alkali metal hydroxide. The size of the polyester particles affects the rate of depolymerization (small particles depolymerize more quickly than larger particles because of larger surface area exposed to the hydroxide). The reaction is typically conducted at about 100°C, under atmospheric conditions, for reaction times in the range of from less than an hour to up to 10 hours, or until most of the polyester depolymerizes. Higher pressures and temperatures may be used, and may reduce the time required to depolymerize the polyester.

The alkali metal hydroxide is typically provided in excess, for example, three moles of alkali metal hydroxide (50% excess) for each mole of polyester, e.g. PET. Alkali metal hydroxide at 100% excess or 150% excess may be employed, but levels of 10% excess to 50% excess, based upon the ratio of moles of hydroxide to moles of polyester or equivalent moles of dicarboxylic acid, are preferred. The amount of hydroxide employed should be sufficient to dissolve the polyester in a reasonable amount of time. The alkali metal hydroxide is, for example, sodium hydroxide or potassium hydroxide or a mixture thereof. Sodium hydroxide is preferred on the basis of cost and availability.

Optionally, a quaternary ammonium hydroxide, for example an unsubstituted or phenyl substituted ($C_{1-4}$) alkyl ammonium hydroxide, preferably tetramethyl ammonium hydroxide or benzyl trimethyl ammonium hydroxide, may be used in combination with the alkali metal hydroxide to increase the rate of the hydrolysis reaction. The quaternary ammonium hydroxide is suitably employed in a small amount, for example, 1 mole percent, based upon total moles of alkali metal hydroxide plus moles of quaternary ammonium hydroxide. Quantities in the range of from 0.1 mole percent to 10 mole percent quaternary ammonium hydroxide, preferably from 0.5 mole percent to 2 mole percent, based upon total moles of alkali metal hydroxide plus moles of quaternary ammonium hydroxide, are suitable for use in the subject invention.

As an alternative, optionally, addition of nonionic surfactant may be used in combination with the alkali metal hydroxide to increase the rate of the hydrolysis reaction. One group of suitable nonionic surfactants have the structural formula

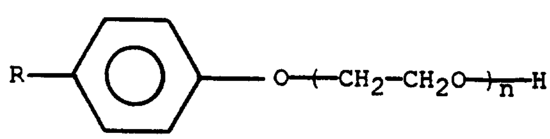

wherein n is an integer of from 1 to 40 and wherein R is a branched alkyl group containing from 6 to 12 carbon atoms, and preferably 8 or 9 carbon atoms. Such nonionic surfactants are sold by Rohm and Haas under the tradename TRITON. For example, octoxynol, Triton X-100, has been found useful in this invention. Other suitable nonionic surfactants include fatty alcohols containing from 5 to 22 carbon atoms, and preferably 10 to 18 carbon atoms. Such nonionic surfactants are marketed by Shell Chemical Company under the tradename NEODOL. For example, Neodol 91-6 would be useful in this invention. Another group of suitable nonionic surfactants includes simple polyethers of ethylene oxide, propylene oxide, and butylene oxide. For example, Polyglycol E200, available from Dow Chemical Company, would be useful in this invention. The nonionic surfactant is optionally employed in a small amount, for example, 0.1 weight percent, based on total amount of polyester, e.g. PET, charged. Amounts in the range of from 0.001 weight percent to 0.5 weight percent nonionic surfactant, preferably from 0.01 weight percent to 0.2 weight percent, based upon total weight of polyester charged, are suitable for use in the subject invention.

To form the hydrolyzed mixture, an amount of liquid sufficient to facilitate the mixing of the components is desirable. Typically, the alkali metal hydroxide is provided in an aqueous solution, or water is added in addition to the alkali metal hydroxide, to facilitate mixing the polyester and alkali metal hydroxide, and the depolymerization products generated, alkali metal salt and glycol. However, a relatively dry hydrolysis process in which the polyester and alkali metal hydroxide are combined, for example, in an extruder, with only a minimal amount of water present, is also envisaged.

In the subject invention, it has been determined that impurities present in the hydrolyzed mixture, which may impart colour in a terephthalic acid product derived from the application of the process to PET polyester, may be oxidized to

insoluble impurities to facilitate their removal. Oxidation may also convert some impurities to water soluble products that are removed from the resulting acid by washing. The oxidizing agent employed in the oxidation step may be selected from air, ozone, chlorine, oxygen, hydrogen peroxide, sodium hypochlorite, and potassium permanganate. Preferably the oxidising agent is selected from air, hydrogen peroxide, ozone, oxygen and mixtures of any two or more thereof; more preferably the oxidising agent is air. The amount of oxidizing agent employed will depend upon the type of agent employed, and should be sufficient to reduce impurities in the dicarboxylic, e.g. terephthalic, acid product to desired levels within a reasonable time period. Air is particularly preferred as the oxidizing agent on the basis of cost and availability.

To facilitate the oxidation process, the amount of water present during the oxidation step should be sufficient to form a solution having in the range of from 5 weight percent to 30 weight percent of alkali metal salt, e.g. alkali metal terephthalate, preferably a 10 weight percent to 20 weight percent solution of alkali metal salt, based upon the total amount of liquid present. If a relatively dry hydrolysis process is used, water may be added as part of the oxidation step. The hydrolyzed mixture may be contacted with the oxidizing agent by any conventional method. The oxidation step is conducted, for example, by sparging the hydrolyzed mixture with air, for from a few minutes to several hours, suitably from 30 minutes to 5 hours, preferably from 1 to 4 hours. Sparging may occur using an air volumetric flow rate in the range of from 1 to 10, preferably 3 to 6, cubic feet per hour (7 to 80 x $10^{-6}$, preferably 2 to 5 x $10^{-5}$, $m^3/s$). Alternatively, the hydrolyzed mixture may be contacted with the oxidizing agent in a thin film reactor, such as a packed column. The resulting oxidized mixture contains insoluble impurities (both organic and inorganic) that are suitably removed by filtration or other conventional means.

The oxidized mixture, after removal of the insoluble impurities, is acidified with a mineral acid to precipitate the resulting acid, e.g. terephthalic acid, in a conventional method. The mineral acid is for example, sulphuric acid, hydrochloric acid, or nitric acid. As an alternative, carbonic acid or carbon dioxide may be used to acidify the oxidized mixture. The dicarboxylic acid may be recovered from the acidified mixture by filtration or some other suitable means, and then washed and dried.

In an optional additional step, diol or glycol, e.g. ethylene glycol, also produced from the hydrolysis reaction, may be recovered by extraction, distillation, or other conventional means. The recovery step may occur at any point in the overall process scheme subsequent to the depolymerization reaction. For PET, recovery of ethylene glycol by extraction with, for example, an alcohol, offers the advantage that it would further reduce the impurities present in the terephthalic acid product, if it occurs prior to precipitation of the terephthalic acid.

Optionally, an alcohol may be combined with, or substituted for the added water, in forming the hydrolyzed mixture. Preferably, for PET, the added alcohol also serves to extract the ethylene glycol produced in depolymerizing the PET. Suitable alcohols include aliphatic or cycloaliphatic alcohols with from one to eight carbon atoms, for example, methanol, ethanol, isopropanol, or butanol, and preferably, for PET, alcohols in which ethylene glycol would be soluble, e.g. isopropanol. If the alcohol is substituted for water, an amount of alcohol sufficient to facilitate mixing the components is desirable. Also, if the alcohol is the sole diluent, and the extraction step occurs prior to oxidation, it is necessary to add another diluent, such as water, to facilitate the oxidation step. The amount of water added to facilitate the oxidation step should be sufficient to form a 5 weight percent to 30 weight percent solution of alkali metal salt, e.g. terephthalate, preferably a 10 weight percent to 20 weight percent solution of alkali metal salt, based upon the total amount of liquid present. For PET, when the ethylene glycol extraction step occurs prior to precipitation of the terephthalic acid, additional impurities may be removed by the alcohol, further improving the quality of the terephthalic acid recovered.

The invention is further illustrated by the following comparative and illustrative Examples.

Example 1

A sodium hydroxide solution was prepared by adding 180.0 g sodium hydroxide to an equal weight of deionized water in a stainless steel beaker. Clear polyethylene terephthalate (PET) flake (288 g) was added to the solution, and the mixture was heated and stirred at reflux for 1.5 hours at 112° to 118°C, then diluted to about 800 ml with water. The mixture was heated for an additional 6 hours at 95° to 100°C, then diluted to 3 litres. The solution soon started to form a precipitate.

About half of the solution (Example 1A) was decanted off and sparged with air for 3 hours. Example 1A was filtered (using Whatman #3 filter paper) to remove 0.138 g insoluble impurities, then acidified to a pH of about 2.5. The precipitated terephthalic acid (TPA) was filtered (using Whatman #1 filter paper), washed, and dried, to produce 100.0 g TPA. The amount of impurities removed from Example 1A was 1.38 x $10^{-3}$ g/g TPA recovered.

The other half of the solution (Example 1B) was refluxed for 3 hours, then filtered (using a coarse wire mesh screen and Whatman #3 filter paper) to remove 27.6 g unreacted PET flake and 0.064 g insoluble impurities. The filtrate was acidified to a pH of about 2.5, and the precipitated TPA was filtered (using Whatman #1 filter paper), washed, and dried. The amount of TPA recovered from Example 1B was 110.1 g. The amount of impurities removed from Example 1B was 0.579 x $10^{-3}$ g/g TPA recovered.

The total yield of TPA from Examples 1A and 1B was 93.3%. To measure the colour of the TPA products, a 12.5 weight percent solution of potassium hydroxide in deionized water was prepared, and 10.00 g of this solution was added to 1.500 g TPA in a vial. The mixture was shaken until dissolved, then an aliquot was placed in a cuvette and inserted in the UV-visible spectrophotometer (Hewlett Packard model 8450A). The absorbance was measured at 350 nm. The higher the absorbance at this wavelength, the more violet/blue light is absorbed and the more yellow the samples appear. Example 1A exhibited an absorbance value of 0.281, while Example 1B exhibited a value of 0.356. These values illustrate the colour improvement that occurs through use of the oxidation step. For reference, virgin TPA exhibits an absorbance of about 0.045. Based upon visual observation, the recycle TPA samples were indistinguishable from virgin TPA.

Example 2

Sodium hydroxide (180 g) deionized water (180 g), and clear PET flake (288 g) were combined as described in Example 1. The mixture was refluxed at 100° to 110°C for 6 hours, with periodic additions of water to keep the mixture stirrable. The mixture was then diluted to about 800 ml, heated to 80° to 90°C, and filtered through a coarse wire mesh screen to remove 24.4 g unreacted PET. The remaining solution began to form a precipitate.

A portion of the solution (Example 2A) was decanted off, filtered (using Whatman #3 filter paper) to remove 0.159 g insoluble impurities, and then acidified to a pH of about 2.5 to precipitate TPA. The TPA precipitate was filtered (using Whatman #1 filter paper), washed, and dried, resulting in a recovery of 159.5 g TPA. The amount of impurities removed from Example 2A was $0.996 \times 10^{-3}$ g/g TPA recovered.

The other portion of the solution (Example 2B) was sparged with air for 4 hours before filtering to remove 0.0867 g insoluble impurities. The filtrate was acidified to a pH of about 2.5 to precipitate TPA, and the TPA precipitate was filtered, washed, and dried, resulting in a recovery of 63.3 g TPA. The amount of impurities recovered from Example 2B was $1.37 \times 10^{-3}$ g/g TPA recovered.

The total recovery of TPA from Examples 2A and 2B was 97.8%.

Example 3

Clear PET flake (288 g), sodium hydroxide (132 g), and deionized water (130 g) were combined in a 1 litre reactor and heated under pressure to 165°C (temperature was reached in 35 minutes). After 3 hours at 165°C, 300 ml of water was charged to the reactor and the temperature brought below 90°C. The suspension was discharged from the reactor, and the reactor flushed twice with 250 ml portions of water. The reactor was then disassembled and any remaining material collected. The combined washings were then heated to about 80°C to dissolve the sodium terephthalate, and the unreacted PET flake was separated by pouring the solution through a 15 mesh screen. The unreacted flake (57.8 g, 20.0% of charge) was washed with water and dried. The sodium terephthalate solution and washings were then sparged with air at a rate of 3 to 6 standard cubic feet per hour ($2.4 \times 10^{-5}$ to $4.7 \times 10^{-5}$ $m^3/s$) for 1 hour. The solution was then filtered (using Whatman #3 filter paper), collecting 0.3224 g impurities. The filtered solution was acidified with 50% sulphuric acid to a pH of 2.0 to 3.0 to precipitate the TPA. The TPA was filtered off, washed and dried. The amount of TPA recovered was 191.1 g or 96.0% of the theoretical amount. The amount of impurities removed was $1.69 \times 10^{-3}$ g/g TPA recovered.

Example 4

Using the method of Example 3, clear PET flake (288 g), sodium hydroxide (130.7 g), potassium hydroxide (1.9 g), and deionized water (130 g) were charged to the reactor. The temperature was brought up to 165°C over 60 minutes, and held at that temperature for 3 hours. After workup, 47.7 g PET flake (16.6% of charge) was recovered. After air oxidation, 0.3465 g impurities were removed and 199.9 g TPA was recovered (96.2% theory) after acidification. The amount of impurities removed was $1.73 \times 10^{-3}$ g/g TPA recovered.

Example 5

Using the method of Example 3, clear PET flake (288 g), sodium hydroxide (126 g), potassium hydroxide (8.4 g), and deionized water (130 g) were charged to the reactor. The reactor temperature was increased to 165°C over 45 minutes, and held at that temperature for three hours. Unreacted PET flake recovered was 42.8 g (14.9% of charge). After air oxidation, 0.3612 g impurities were removed, and 203.6 g TPA (96.0% of theory) were recovered. The amount of impurities removed was $1.77 \times 10^{-3}$ g/g TPA recovered, and the terephthalic acid exhibited an absorbance of 0.284.

Example 6, according to the invention

Using the method of Example 3, clear PET flake (288 g), sodium hydroxide (130.7 g), 25 weight percent aqueous solution of tetramethyl ammonium hydroxide (12.0 g), and deionized water (130 g) were charged to the reactor. The reactor temperature was increased to 165°C over 30 minutes, and held at that temperature for 3 hours. Unreacted PET flake recovered was 39.1 g (13.6% of charge). After air oxidation, 0.3644 g impurities were removed, and 207.8 g TPA (96.6% of theory) were recovered. The amount of impurities removed was 1.75 x $10^{-3}$ g/g TPA recovered.

The amount of unreacted PET in Example 6 (13.6% of charge) is significantly less than the amount for Example 3 (20% of charge), although the reaction temperatures and times were equivalent. This indicates that the tetramethyl ammonium hydroxide increased the rate of the depolymerization reaction.

Example 7

Clear PET flake (288 g), sodium hydroxide (130.7 g), potassium hydroxide (1.9 g), and deionized water (130 g) were combined in a 1 litre reactor and heated under pressure to 100°C. Once the reactor reached 100°C, 250 ml isopropanol was added through an injection port. The mixture was then heated to 150°C over 35 minutes. The mixture was held at 150°C for 3 hours, then cooled to below 80°C. The reactor was then emptied and rinsed with isopropanol. The slurry was filtered and the filtercake washed with isopropanol.

The filtercake was dissolved in 2.5 litres hot water and poured through a 15 mesh screen to remove unreacted PET (14.4 g or 5.0% of charge was recovered). The filtrate was sparged with air for 1 hour at 3 to 6 standard cubic feet per hour (2.4 x $10^{-5}$ to 4.7 x $10^{-5}$ $m^3/s$), then filtered (using Whatman #3 filter paper), removing 0.2236 g impurities. The filtrate was acidified to pH 2.5 to precipitate the terephthalic acid, which was filtered, washed and dried. The yield of TPA was 230.5 g (97.4% of theory), and it exhibited an absorbance of 0.299.

The combined isopropanol fractions were concentrated on a rotary evaporator to yield 88.9 g of rose-coloured ethylene glycol with visible contamination by glue, etc. A portion (83.1 g) of this solution was vacuum distilled at 80°C to yield 53.0 g (64% theory) ethylene glycol and leaving 25.1 g residue (tan paste) in the distillation pot.

For this procedure, the amount of impurities removed was the amount removed by filtration, plus a portion of the residue remaining after ethylene glycol recovery.

Example 8, according to the invention

Using the method of Example 3, clear PET flake (288 g), sodium hydroxide (132 g), Triton X-100, a nonionic surfactant (0.28 g), and deionized water (130 g) were charged to the reactor. The reactor temperature was increased to 165°C over 35 minutes, and held at that temperature for 3 hours. Unreacted PET flake recovered was 34.5 g (12.0% of charge). After air oxidation, 0.4582 g impurities were removed, and 209.9 g TPA (97.7% of theory) were recovered. The amount of impurities removed was 2.18 x $10^{-3}$ g/g TPA recovered.

The amount of unreacted PET in Example 8 (12.0% of charge) is significantly less than the amount for Example 3 (20% of charge), although the reaction temperatures and times were essentially equivalent. This indicates that the nonionic surfactant increased the rate of the depolymerization reaction.

In the above examples, Examples 6 and 8 are of the invention as claimed, and Examples 1B and 2A are comparative examples. The table below summarizes the results of the amount of impurities removed by the various methods described, and the colour of the resulting terephthalic acid product, where measured. The addition of an aeration step clearly results in an increased removal of impurities from the TPA recycle stream, thereby providing a higher quality TPA product. The addition of a quaternary ammonium hydroxide, a nonionic surfactant or an alcohol in the first (basic hydrolysis) step provide further advantages.

| Example | Method | Impurities Recovered * g/g TPA | Colour Asorbance * (@ 350 nm) |
|---|---|---|---|
| 1A not claimed | Heating (7.5 hr) + Aeration (3 hr) | $1.38 \times 10^{-3}$ | 0.281 |
| 1B comparative | Heating (10.5 hr) | $0.579 \times 10^{-3}$ | 0.356 |
| 2A comparative | Heating (6 hr) | $0.996 \times 10^{-3}$ | - |
| 2B not claimed | Heating (6 hr) + Aeration (4 hr) | $1.37 \times 10^{-3}$ | - |
| 3 not claimed | Heating (3 hr) + Aeration (1 hr) | $1.69 \times 10^{-3}$ | - |
| 4 not claimed | Heating (3 hr) + Aeration (1 hr) | $1.73 \times 10^{-3}$ | - |
| 5 not claimed | Heating (3 hr) + Aeration (1 hr) | $1.77 \times 10^{-3}$ | 0.284 |
| 6 | Heating (3 hr) + Aeration (1 hr) | $1.75 \times 10^{-3}$ | - |
| 7 not claimed | Heating (3 hr) + Aeration (1 hr) | - | 0.299 |
| 8 | Heating (3 hr) + Aeration (1 hr) | $2.18 \times 10^{-3}$ | - |

* where measured

## Claims

1. A process for recovering dicarboxylic acid from a polyester feed stream which comprises:

   (a) contacting the polyester with an alkali metal hydroxide and a quaternary ammonium hydroxide or a nonionic surfactant and, if necessary, water to form a hydrolyzed mixture containing an alkali metal carboxylic acid salt and a diol or glycol,
   (b) oxidizing the hydrolyzed mixture through addition of an oxidizing agent, to form an oxidized mixture containing insoluble impurities,
   (c) removing the insoluble impurities from the oxidized mixture,
   (d) acidifying the oxidized mixture to form an acidified mixture from which the dicarboxylic acid is precipitated, and
   (e) recovering dicarboxylic acid from the acidified mixture.

2. A process as claimed in claim 1, wherein the polyester is polyethylene terephthalate and the dicarboxylic acid is terephthalic acid.

3. A process as claimed in claim 1 or claim 2, wherein the oxidizing agent is selected from the group consisting of air, hydrogen peroxide, ozone, oxygen, and mixtures of any two or more thereof.

4. A process as claimed in claim 3, wherein the oxidising agent is air.

5. A process as claimed in any one of claims 1 to 4, wherein the step of oxidizing the hydrolyzed mixture is conducted by sparging the hydrolyzed mixture with air.

6. A process as claimed in any one of claims 1 to 5, wherein an alcohol is included as an additional component to form the hydrolyzed mixture.

## Patentansprüche

1. Verfahren zur Rückgewinnung von Dicarbonsäure aus einem Polyester-Einsatzstrom, welches Verfahren umfaßt:

   (a) Kontaktieren des Polyesters mit einem Alkalimetallhydroxid und einem quaternären Ammoniumhydroxid oder einem nichtionischen grenzflächenaktiven Mittel und erforderlichenfalls Wasser zur Ausbildung eines hydrolysierten Gemisches, das ein Alkalimetall-Carbonsäuresalz und ein Diol oder Glycol enthält,

(b) Oxidieren des hydrolysierten Gemisches durch Zusetzen eines Oxidationsmittels zur Ausbildung eines oxidierten Gemisches mit einem Gehalt an unlöslichen Verunreinungen,
(c) Abtrennen der unlöslichen Verunreinigungen aus dem oxi- dierten Gemisch,
(d) Ansäuern des oxidierten Gemisches zur Ausbildung eines angesäuerten Gemisches, aus dem die Dicarbonsäure ausgefällt wird, und
(e) Gewinnen der Dicarbonsäure aus dem angesäuerten Gemisch.

**2.** Verfahren nach Anspruch 1, worin der Polyester Polyethylenterephthalat ist und die Dicarbonsäure Terephthalsäure ist.

**3.** Verfahren nach Anspruch 1 oder Anspruch 2, worin das Oxidationsmittel von der aus Luft, Wasserstoffperoxid, Ozon, Sauerstoff und Gemischen von beliebigen zwei oder mehreren hievon bestehenden Gruppe ausgewählt wird.

**4.** Verfahren nach Anspruch 3, worin das Oxidationsmittel Luft ist.

**5.** Verfahren nach einem der Ansprüche 1 bis 4, worin die Stufe des Oxidierens des hydrolysierten Gemisches durch Spülen des hydrolysierten Gemisches mit Luft ausgeführt wird.

**6.** Verfahren nach einem der Ansprüche 1 bis 5, worin ein Alkohol als eine zusätzliche Komponente zur Ausbildung des hydrolysierten Gemisches eingeschlossen wird.

**Revendications**

**1.** Procédé de récupération de l'acide dicarboxylique à partir d'un courant d'alimentation en polyester, caractérisé en ce qu'il comprend :

(a) la mise en contact du polyester avec un hydroxyde de métal alcalin et un hydroxyde d'ammonium quaternaire, ou un surfacique ou tensioactif non ionique et, si cela se révèle nécessaire, de l'eau pour former un mélange hydrolysé contenant un sel de métal alcalin de l'acide carboxylique et un diol ou glycol,
(b) l'oxydation du mélange hydrolysé par l'addition d'un agent oxydant, pour former un mélange oxydé contenant des impuretés insolubles,
(c) l'élimination des impuretés insolubles du mélange oxydé,
(d) l'acidification du mélange oxydé pour former un mélange acidifié à partir duquel on précipite l'acide dicarboxylique, et
(e) la récupération de l'acide dicarboxylique à partir du mélange acidifié.

**2.** Procédé suivant la revendication 1, caractérisé en ce que le polyester est le poly(téréphtalate d'éthylène) et l'acide dicarboxylique est l'acide téréphtalique.

**3.** Procédé suivant la revendication 1 ou la revendication 2, caractérisé en ce que l'on choisit l'agent oxydant dans le groupe formé par l'air, le peroxyde d'hydrogène, l'ozone, l'oxygène et des mélanges de n'importe lesquels de deux ou plus de deux d'entre eux.

**4.** Procédé suivant la revendication 3, caractérisé en ce que l'agent oxydant est l'air.

**5.** Procédé suivant l'une quelconque des revendications 1 à 4, caractérisé en ce que l'étape d'oxydation du mélange hydrolysé s'entreprend en injectant de l'air dans le mélange hydrolysé.

**6.** Procédé suivant l'une quelconque des revendications 1 à 5, caractérisé en ce que l'on ajoute un alcool à titre de composant supplémentaire pour former le mélange hydrolysé.